# EUROPEAN PATENT APPLICATION

(11) **EP 3 712 127 A1**
(43) Date of publication of application: **23.09.2020**
(21) Application number: 19164703.1
(22) Date of filing: 22.03.2019
(51) Int. Cl.: C07C 259/06, A61P 35/00

(54) **NOVEL INHIBITORS OF HISTONE DEACETYLASE 10**

(71) Applicant: Deutsches Krebsforschungszentrum, 69120 Heidelberg (DE)
(72) Inventor: Miller, Aubry K., 69126 Heidelberg (DE); Steimbach, Raphael, 69124 Heidelberg (DE); Géraldy, Magalie, 51240 Mairy sur Marne (FR)
(74) Representative: Virnekäs, Bernhard

(57) **Abstract**

The present invention relates to novel inhibitors of histone deacetylase 10 (HDAC10), novel pharmaceutical compositions comprising such inhibitors, and to novel methods of treating cancer using such novel inhibitors.

## Description

The present invention relates to novel inhibitors of histone deacetylase 10 (HDAC10), novel pharmaceutical compositions comprising such inhibitors, and to novel methods of treating cancer using such novel inhibitors.

### BACKGROUND

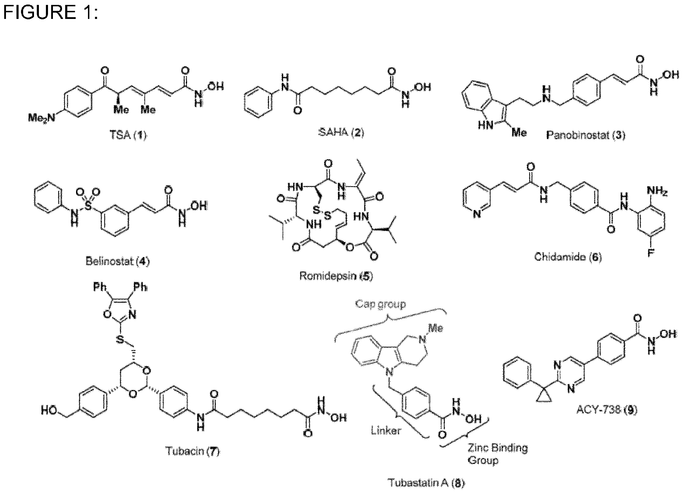

The discovery of histone deacetylase 1 (HDAC1) by Taunton and Schreiber in 1996¹ provided the long sought-after enzyme target for substances like trichostatin A (TSA (1), Figure 1) and suberanilohydroxamic acid (SAHA (2)). At the time, 1 and 2 were reported to increase histone lysine acetylation levels, thereby inducing cellular differentiation, but their mechanism(s) of action were unknown.² Taunton and Schreiber's disclosure launched a now two-decade's long effort to discover inhibitors of HDACs, currently a family of 18 functionally related isozymes.³⁻⁴ During this time it has also become clear that HDACs have a broader role than catalyzing the hydrolysis of acetylated histone lysines: not only do they act in both the nucleus and the cytoplasm, but they catalyze the removal of *acyl* groups from a variety of different proteins.⁵⁻⁹ The 18 HDACs are grouped into four different classes based on homology to their yeast orthologs as follows: Class I (HDAC1, -2, -3, and -8); Class II, which is subdivided into Class IIA (HDAC4, -5, -7, and -9) and Class IIB (HDAC6 and -10); Class III (sirtuin1-7); and Class IV (HDAC11).¹⁰ While Classes I, IIA-B, and IV are Zn²⁺-dependent amidohydrolases, the Class III sirtuins are mechanistically distinct NAD+-dependent enzymes. For this reason, the sirtuins are often considered separately in discussions of "HDAC inhibitors". Currently, there are four HDAC inhibitor drugs approved in the U.S. (**2-5**), one in China (**6**), many other candidates undergoing clinical trials, and dozens of reported inhibitors (Figure 1). The approved drugs are used as anti-cancer agents, but HDAC inhibitors are also investigated in the treatment of autoimmune disorders, and neurodegeneration.³ Clinically used pan-HDAC inhibitor drugs (e.g. **2-5**) can cause severe side effects, caused in part by their lack of selectivity. More isozyme-selective inhibitors are expected to overcome these liabilities and are likely to improve the clinical value of this target class.¹⁰⁻¹¹ Moreover, the development of isozyme-selective chemical probes will be critical to further disentangle the biological role(s) of individual HDAC isozymes.¹² To date, the most significant focus and success in the development of selective inhibitors has been with the Class I enzymes, where selective inhibitors of HDAC1/2, HDAC3, and HDAC8 have been disclosed, and with the Class IIB enzyme HDAC6.³ Far fewer selective inhibitors of the Class IIA, HDAC10 or HDAC11 subtypes have been reported.¹³⁻¹⁷ In 2003, tubacin (**7**) was described as the first selective HDAC6 inhibitor (Figure 1).¹⁸ In the intervening years, many additional HDAC6 inhibitors with good selectivity profiles have been described, the most well-known (besides tubacin) being tubastatin A (**8**).¹⁹ Indeed, both **7** and **8,** along with ACY-738 (**9**)²⁰, are designated as HDAC6 chemical probes in the Chemical Probes Portal.²¹ Most HDAC6-selective inhibitors, like **8** and **9,** achieve selectivity over Class I enzymes by incorporating a relatively bulky phenyl hydroxamate "linker" moiety in addition to a "cap group" that can make specific interactions with the HDAC6 protein surface (see **8,** Figure 1).

HDAC10 was first isolated in 2002 and annotated as a Class IIB HDAC based on its high similarity to HDAC6.²²⁻²⁴ Like HDAC6, HDAC10 appears to localize to both the nucleus and cytoplasm, and has been reported to interact with proteins having a variety of functions including transcription factors²⁵ and cyclins,²⁶ and to play a prominent role in homologous recombination²⁷ and Hsp-mediated VEGFR regulation.²⁸ While some clinical correlation studies have indicated an apparent tumor-suppressor function for HDAC10,²⁹⁻³³ a number of other studies highlight HDAC10 as a potential cancer drug target.³⁴⁻³⁷ In one study, high HDAC10 expression levels were found to correlate with poor clinical outcome for advanced stage 4 neuroblastoma patients who received chemotherapy.³⁷ Consistent with these findings, HDAC10 depletion in neuroblastoma cells interrupts autophagic flux and sensitizes cells for chemotherapy, and enforced HDAC10 expression protects neuroblastoma cells against doxorubicin treatment.³⁸

Additionally, it has been found that HDAC10 is involved in the regulation of PD-L1 Expression and immune tolerance mediated by antigen presenting cells (APCs).⁴² It could be shown that in macrophages isolated from HDAC10 knock-out mice exhibited an increased expression of MHC II molecules and a decreased expression of PD-L1. In an in vivo model, tumor growth was delayed in HDAC10 knock-out mice when compared to wild-type mice. Thus, the disruption of the HDAC10/PD-L1 axis could provide a novel target for cancer immunotherapy.

Recently, Christianson and co-workers solved the x-ray crystal structure of zebrafish HDAC10 (*z*HDAC10).⁶ They elegantly demonstrated that both the zebrafish and human HDAC10 (*h*HDAC10) enzymes are highly active polyamine deacetylases (PDAC), while being poor lysine deacetylases. Therefore, it appears as if HDAC10 may not act on proteins, but on polyamine metabolites, e.g. spermidine and putrescine. Two specific structural features near the active site of the enzyme(s) were identified as being responsible for PDAC activity. First, the negatively charged Glu272 (*h*HDAC10 numbering) amino acid was demonstrated to be a gatekeeper residue, which establishes specificity for cationic polyamine substrates over acetylated lysines. In all other HDAC isozymes except the first catalytic domain of HDAC6, this amino acid is hydrophobic, usually a leucine. Second, the L1 loop of HDAC10 contains a two-residue insertion relative to HDAC6 in both zebrafish and humans. Christianson et al. found that, in zHDAC10, these inserted residues plus a two-residue mutation create a unique 310-helix that constricts the active site, making an acetylated lysine side chain, but not an acetylated polyamine, too short to reach the active site zinc atom. In *h*HDAC10, these four residues, numbered 21-24, are Pro-Glu-Cys-Glu. Both an E272L HDAC10 mutant and a mutant lacking the two-residue loop insertion were found to have increased HDAC activity and diminished PDAC activity in enzymatic assays. Interestingly, this model suggests that bulky (e.g. **8**) Class inhibitors cannot fit into the constricted binding pocket of HDAC10 without significant movement of the L1 loop. The fact that an E272L mutation alone is sufficient to enable deacetylation of lysines, however, suggests that the L1 loop may have this flexibility.

After it had been found that some known HDAC inhibitors bind HDAC10 as well,³⁸⁻⁴⁰ Géraldy et al. could show an unexpectedly potent HDAC10 binding of tubastatin A, which had previously been described as a highly selective HDAC6 inhibitor.⁴¹ Analysis of a targeted selection of tubastatin A derivatives revealed that a basic amine in the cap group was required for strong HDAC10, but not HDAC6, binding. Only potent HDAC10 binders mimicked HDAC10 knockdown by causing dose-dependent accumulation of acidic vesicles in the BE(2)-C neuroblastoma cell line. Docking of inhibitors into human HDAC10 homology models indicated that a hydrogen-bond between a basic cap group nitrogen and the HDAC10 gatekeeper residue Glu272 was responsible for potent HDAC10 binding.

While the work of Géraldy et al. has shown the possibility of identifying potent HDAC10 inhibitor, there was still an unmet need for finding such a potent inhibitor, which simultaneously was no longer significantly binding to HDAC6 and/or to other HDACs.

### SUMMARY OF THE INVENTION

The present invention is based on the surprising observation that variants of suberanilohydroxamic acid (SAHA), which comprise the substitution of a particular methylene group by an amino group, result in the formation of potent and specific HDAC10 inhibitors.

Thus, in a first aspect, the present invention relates to an HDAC10 inhibitor of Formula (I)

CAP-(CR^{y}R^{y'})ₙ-NR²-CR³R^{3'}-CR⁴R^{4'}-CR⁵R^{5'}-ZBD (I)

wherein:
CAP is a capping group selected from the group of aryl-X- and heteroaryl-X-, wherein X is absent or is selected from -C(=O)-NR¹-, -NR-C(=O)-, -S(=O)₂-NR¹-,-NR-S(=O)₂-, -S(=O)(=NR)-NR¹-, -NR-S(=O)(=NR)-, -C(=NR)-, -O-, -S-, -S(=O)-,-S(=O)₂-, and -C(=O)-,
wherein
R and R¹ are each independently a residue selected from H and a substituent selected from linear or branched C₁₋₄-alkyl, cyclopropyl, benzyl, aryl and heteroaryl, wherein said substituent is optionally further substituted, in particular by a further substituent selected from the list of -F, -OH, -OR, and -NR₂;
n is an integer selected from 1, 2 and 3;
y is an integer taking the values from the range of 1 to n;
ZBD is a zinc-binding domain selected from the group of:
   -C(=O)-NH-OH, -C(=N-OH)-C(=O)NH-R⁶, -C(=O)CF₃, -C(=O)-NH-C₆H₄-o-NH₂,-C(=O)CH₂SH, -SH, -C(=NH)-NH-OH, and -C(=N-OH)-NH₂, wherein R⁶ is a residue selected from H, linear or branched C₁₋₄-alkyl,-cyclopropyl, and benzyl;
   and
   R² is a residue selected from H and a substituent selected from linear or branched C₁₋₄-alkyl, cyclopropyl, benzyl, aryl and heteroaryl, wherein said substituent is optionally further substituted, in particular by a further substituent selected from the list of -F, -OH, -OR, and -NR₂, and R¹, each R^{y}, each R^{y'}, R³, R^{3'}, R⁴, R^{4'}, R⁵, and R^{5'} are independently selected from residues H, CH₃, F, CFH₂, CF₂H and CF₃, provided that in total not more than three of said residues are different from -H.

In a second aspect, the present invention relates to a pharmaceutically acceptable salt form of the HDAC10 inhibitor of the present invention.

In a third aspect, the present invention relates to a pharmaceutical composition comprising an HDAC10 inhibitor of the present invention, or a pharmaceutically acceptable salt form of an HDAC10 inhibitor of the present invention.

In a fourth aspect, the present invention relates to an HDAC10 inhibitor of the present invention, a pharmaceutically acceptable salt form of an HDAC10 inhibitor of the present invention or a pharmaceutical composition of the present invention, for use in the treatment of cancer.

In a fifth aspect, the present invention relates to a method of treating cancer, comprising the step of administering an HDAC10 inhibitor of the present invention, a pharmaceutically acceptable salt form of an HDAC10 inhibitor of the present invention, or a pharmaceutical composition of the present invention to a patient suffering from said cancer.

### FIGURES

**Figure 1** shows the structures of different HDAC inhibitors.

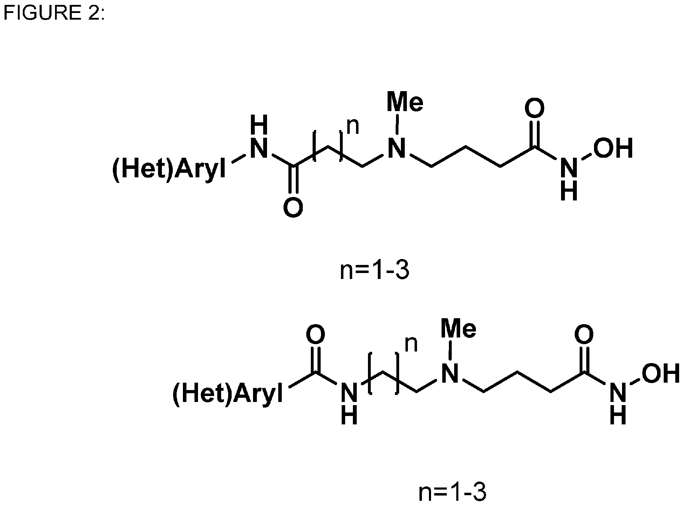

**Figure 2** shows the structures of different HDAC10 inhibitors of the present invention.

### DETAILED DESCRIPTION OF THE INVENTION

In a first aspect, the present invention relates to an HDAC10 inhibitor of Formula (**I**)

CAP-(CR^{y}R^{y'})ₙ-NR²-CR³R^{3'}-CR⁴R^{4'}-CR⁵R^{5'}-ZBD (**I**)

wherein:
CAP is a capping group selected from the group of aryl-X- and heteroaryl-X-, wherein X is absent or is selected from -C(=O)-NR¹-, -NR-C(=O)-, -S(=O)₂-NR¹-,-NR-S(=O)₂-, -S(=O)(=NR)-NR¹-, -NR-S(=O)(=NR)-, -C(=NR)-, -O-, -S-, -S(=O)-,-S(=O)₂-, and -C(=O)-,
wherein
R and R¹ are independently a residue selected from H and a substituent selected from linear or branched C₁₋₄-alkyl, cyclopropyl, benzyl, aryl and heteroaryl, wherein said substituent is optionally further substituted, in particular by a further substituent selected from the list of -F, -OH, -OR, and -NR₂;
n is an integer selected from 1, 2 and 3;
y is an integer taking the values from the range of 1 to n;
ZBD is a zinc-binding domain selected from the group of:
   -C(=O)-NH-OH, -C(=N-OH)-C(=O)NH-R⁶, -C(=O)CF₃, -C(=O)-NH-C₆H₄-o-NH₂,-C(=O)CH₂SH, -SH, -C(=NH)-NH-OH, and -C(=N-OH)-NH₂, wherein R⁶ is a residue selected from H, linear or branched C₁₋₄-alkyl,-cyclopropyl, and benzyl;
   and
   R² is a residue selected from H and a substituent selected from linear or branched C₁₋₄-alkyl, cyclopropyl, benzyl, aryl and heteroaryl, wherein said substituent is optionally further substituted, in particular by a further substituent selected from the list of -F, -OH, -OR, and -NR₂, and R¹, each R^{y}, each R^{y'}, R³, R^{3'}, R⁴, R^{4'}, R⁵, and R^{5'} are independently selected from residues H, CH₃, F, CFH₂, CF₂H and CF₃, provided that in total not more than three of said residues are different from -H.

The present invention is intended to include all isotopes of atoms occurring on the present compound. Isotopes are atoms having the same atomic number but different mass numbers. By way of general example and without limitation, isotopes of hydrogen include deuterium and tritium, in particular deuterium. Isotopes of carbon include ¹²C and ¹⁴C.

In the context of the present invention, the term "aryl" is intended to mean a ring or ring system being part of any stable monocyclic or polycyclic system, where such ring or ring system has between 3 and about 20 carbon atoms, but has no heteroatom, and where the ring or ring system consists of an aromatic moiety as defined by the "(4n+2) π electron rule". For the sake of clarity, if a substituent is a polycyclic system wherein one ring or ring system comprised in said polycyclic system consists of an aromatic moiety as defined herein, then such substituent will be referred to as "aryl", if substitution occurs via said aromatic moiety. This includes, but is not limited to, phenyl and fused benzene ring systems, for example, naphthalene, anthracene, or phenanthrene ring systems, or, for example, a benzene ring fused to one or more cycloalkyl moieties to form, for example, indanyl, fluorenyl or tetrahydronaphthyl (tetralin), or fused to one or more heterocycloalklyl rings, e.g. as in indolenyl, provided, however, that in each such case, such fused system is linked as a substituent via the aromatic moiety. As used herein, the term "heteroaryl" refers to a ring or ring system being part of any stable mono- or polycyclic system, where such ring or ring system has between 3 and about 20 atoms, which ring or ring system consists of an aromatic moiety as defined by the "(4n+2) π electron rule" and which contains carbon atoms and one or more nitrogen, sulfur, and/or oxygen heteroatoms. For the sake of clarity, if a substituent is a polycyclic system wherein one ring or ring system comprised in said polycyclic system consists of an aromatic moiety containing a heteroatom as defined herein, then such substituent will be referred to as "heteroaryl", if substitution occurs via the aromatic moiety containing the heteroatom. In certain embodiments, the total number of N, S and O atoms in the heteroaryl is between 1 and about 4. In certain embodiments, the total number of S and O atoms in the aromatic heteroaryl is not more than 1. In certain embodiments, a nitrogen in the heterocycle may be quaternized or oxidized to an N-oxide. Examples of heteroaryls include, but are not limited to, pyrrolyl, pyrazolyl, imidazolyl, indolyl, benzimidazolyl, furanyl, benzofuranyl, thiophenyl, benzothiophenyl, pyridinyl, pyrimidinyl, pyrazinyl, triazinyl, quinolinyl, quinazolinyl. Also included in the term heteroaryl are fused heteroaryls containing, for example, the above heteroaryls fused to cycloalkyls or heterocycloalkyls (provided, in each case, that such fused system is linked as a substituent via the aromatic moiety containing at least one heteroatom).

In the context of the present invention, the term "substituted" is intended to indicate that one or more hydrogens on the atom or group indicated in the expression using "substituted" is replaced with a selection from the indicated group(s), provided that the indicated atom's normal valency, or that of the appropriate atom of the group that is substituted, is not exceeded, and that the substitution results in a stable compound. The terms "substituted or unsubstituted" or "optionally substituted" are intended to mean that a given compound, or substructure of a compound, is either unsubstituted, or substituted, as defined herein, with one or more substituents as indicated.

In one embodiment of the present invention, the compound of the invention has a purity of more than 90%, more than 95%, more than 98%, or more than 99%. The compound may exist in one or more crystalline forms, including two or more polymorphic forms, and may exist as a dry solid or as a solvate including a defined amount of a solvent, including a hydrate including defined amounts of water.

In another embodiment, the compound of the invention is the planned and deliberate product of a synthetic chemistry scheme, i.e., produced by specific and planned chemical processes conducted in reaction vessels, and not by degradation, metabolism or fermentation, or produced as impurity or by-product in the synthesis of other compounds.

In certain embodiments, the compound of the invention is purified or isolated, e.g., to have a purity of at least 80%, preferably at least 90%, more preferably at least 95%, such as at least 97%, at least 98% or even at least 99%. Purity, as used herein, can refer to either absolute or relative purity. Absolute purity refers to the amount of the compound of the invention obtained as the product of a synthetic chemistry scheme, either before or after one or more purification steps. Relative purity refers to the amount of the compound of the invention relative to one or more impurities such as by-products, degradation products (e.g., metabolites, products of oxidation or hydrolysis, etc.) and/or compounds that degrade to form the compound of the invention (e.g., precursors or prodrugs), e.g., that may be present in the product of a synthetic chemistry scheme. Thus, absolute purity refers to the amount of a compound relative to all others, while relative purity is generally unaffected by the addition of unrelated compounds, such as excipients, stabilizers, or other medicaments for conjoint administration. Purity can be assessed based upon weight, volume or molar ratios of one compound relative to others. Purity can be measured by a variety of analytical techniques, including elemental abundance, UV-visible spectrometry, HPLC, GC-MS, NMR, mass spectrometry, and thin layer chromatography, preferably by HPLC, GC-MS, or NMR.

In particular embodiments, CAP is a capping group selected from the group of: aryl-C(=O)-NH-, heteroaryl-C(=O)-NH-, and aryl-NH-C(=O)-, and heteroaryl-NH-C(=O)-.

In particular such embodiments, CAP is phenyl-NH-C(=O)- or phenyl- C(=O)-NH-, particularly phenyl-NH-C(=O)-.

In particular embodiments, n is 2.

In particular embodiments, R² is a residue selected from H, methyl, ethyl, n-propyl, i-propyl, n-butyl, cyclopropyl, and benzyl.

In particular embodiments, each R^{y}, each R^{y'}, R³, R^{3'}, R⁴, R^{4'}, R⁵, and R^{5'} are each -H.

In particular embodiments, ZBD is -C(=O)-NH-OH.

In particular embodiments, where CAP is phenyl-NH-C(=O)-, n is 2, each R^{y}, each R^{y'}, R³, R^{3'}, R⁴, R^{4'}, R⁵, and R^{5'} are each -H and ZBD is -C(=O)-NH-OH, R² is different from -H.

In particular embodiments, the HDAC10 inhibitor is selected from the group of DKFZ711, DKFZ714, DKFZ715, DKFZ716, DKFZ717, DKFZ718, DKFZ724, and DKFZ728.

In a second aspect, the present invention relates to a pharmaceutically acceptable salt form of the HDAC10 inhibitor of the present invention.

As used herein, "pharmaceutically acceptable salts" refers to derivatives of the disclosed compound wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. Lists of suitable salts are found in Remington's Pharmaceutical Sciences, 18th ed., Mack Publishing Company, Easton, PA, 1990, p. 1445, or in PH Stahl und CG Wermuth (eds.), Handbook of Pharmaceutical Salts: Eigenschaften, Auswahl und Verwendung , Weinheim / Zürich: Wiley-VCH / VHCA, 2002, the disclosure of which is hereby incorporated by reference. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, palmoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from a parent compound which contains a basic or acidic moiety by conventional chemical methods. Generally, such salts can be prepared by reacting the free acid or base forms of the compound with a stoichiometric amount of the appropriate base or acid in water or in an organic solvent, or in a mixture of the two; generally, non-aqueous media like ether, EtOAc, ethanol, isopropanol, or acetonitrile are preferred.

Any salt that retains the desired biological activity of the compound contained herein and that exhibits minimal or no undesired or toxicological effects is intended for inclusion here. Pharmaceutically acceptable salts include those derived from pharmaceutically acceptable organic or inorganic acids and bases. Non-pharmaceutically acceptable acids and bases also find use herein, as for example, in the synthesis and/or purification of the compound of interest. Thus, all "salts" are also encompassed within the scope of the instant invention.

Non-limiting examples of suitable salts include those derived from inorganic acids, such as, for example, hydrochloric acid, hydrobromic acid, sulfuric acid, phosphoric acid, nitric acid, bicarbonic acid, carbonic acid; and salts formed with organic acids, such as, for example, formic acid, acetic acid, oxalic acid, tartaric acid, succinic acid, malic acid, malonic acid, ascorbic acid, citric acid, benzoic acid, tannic acid, palmoic acid, alginic acid, polyglutamic acid, tosic acid, methanesulfonic acid, naphthalenesulfonic acid, naphthalenedisulfonic acid, α-ketoglutaric acid, β-glycerophosphoric acid and polygalacturonic acid.

Suitable salts obtained by reacting an acidic compound with a base include those derived from alkali metals such as lithium, potassium and sodium, from alkaline earth metals such as calcium and magnesium, as well as from other acids well known to those of skill in the pharmaceutical art. Other suitable salts include those derived from metal cations such as zinc, bismuth, barium, or aluminum, or with a cation formed from an amine, such as ammonia, N,N-dibenzylethylene-diamine, D-glucosamine, tetraethylammonium, or ethylenediamine. Moreover, suitable salts include those derived from a combination of acids and bases, such as, for example, a zinc tannate salt.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

In particular embodiments, the HDAC10 of the present invention is reacted with an acid selected from the group of: hydrochloric, hydrobromic, sulfuric, sulfamic, phosphoric, nitric; acetic, propionic, succinic, glycolic, stearic, lactic, malic, tartaric, citric, ascorbic, palmoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic acid, in particular hydrochloric acid.

In a third aspect, the present invention relates to a pharmaceutical composition comprising an HDAC10 inhibitor of the present invention, or a pharmaceutically acceptable salt form of an HDAC10 inhibitor of the present invention.

The compound of this invention can be formulated and administered to treat individuals in need by any means that produces contact of the active ingredient with the agent's site of action, such as a cell, in the body of an individual. It can be administered by any conventional means available for use in conjunction with pharmaceuticals, either as individual therapeutic active ingredients or in a combination of therapeutic active ingredients. It can be administered alone, but are generally administered with a pharmaceutically acceptable diluent, excipient or carrier selected on the basis of the chosen route of administration and standard pharmaceutical practice.

A pharmaceutical composition comprising less than a therapeutically effective amount of the compound described above, or a prodrug thereof, may also be used, such as when used in combination with another pharmaceutical composition, such as an anti-cancer agent, so that such combination is therapeutically effective, or may be useful for prophylactic treatment.

Pharmaceutical compositions for use in accordance with the present invention may be formulated in conventional manner using one or more pharmaceutically acceptable diluents, excipients or carriers. The pharmaceutical compositions of the invention can be formulated for a variety of routes of administration, including systemic and topical or localized administration. Techniques and formulations generally may be found in Remington's Pharmaceutical Sciences, Meade Publishing Co., Easton, PA. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, capsules, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intrarectally, for example, as a pessary, cream or foam. In certain embodiments, the pharmaceutical preparations may be non-pyrogenic, i.e., do not substantially elevate the body temperature of a patient.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of inhibitor which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association the compound of the present invention with the carrier and, optionally, one or more accessory ingredients. In general, the formulations are prepared by uniformly and intimately bringing into association the compound of the present invention with liquid carriers, or finely divided solid carriers, or both, and then, if necessary, shaping the product.

For systemic administration, injection is preferred, including intramuscular, intravenous, intraperitoneal, and subcutaneous (i.m., i.v., i.p., and s.c. respectively). The phrases "systemic administration", "administered systemically", "peripheral administration", and "administered peripherally" as used herein mean the administration of a compound, drug or other material other than directly into the central nervous system, such that it enters the patient's system and, thus, is subject to metabolism and other like processes, for example, subcutaneous administration.

For injection, the pharmaceutical compositions of the invention can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

Pharmaceutical compositions of the invention may be formulated to be suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of the compound of the present invention as an active ingredient. The compound of the present invention may also be administered as a bolus, electuary or paste.

In formulating the pharmaceutical compositions of the invention in solid dosage forms for oral (p.o.) administration (capsules, tablets, pills, dragees, powders, granules and the like), the compound of the invention as active ingredient is mixed with one or more pharmaceutically acceptable carriers, such as sodium citrate or dicalcium phosphate, and/or any of the following: (1) fillers or extenders, such as starches, lactose, sucrose, glucose, mannitol, and/or silicic acid; (2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, high molecular weight polyethylene glycols, and the like.

Gelatin capsules contain the compound of the present invention as active ingredient and powdered carriers, such as lactose, starch, cellulose derivatives, magnesium stearate, stearic acid, and the like. Similar carriers can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract. Solid compositions of a similar type are also employed as fillers in soft and hard-filled gelatin capsules; preferred materials in this connection also include lactose or milk sugar as well as high molecular weight polyethylene glycols. A preferred formulation is a solution or suspension in an oil, for example olive oil, Miglyol, or Capmul, in a soft gelatin capsule. Antioxidants may be added to prevent long-term degradation as appropriate.

A tablet may be made by compression or molding, optionally with one or more accessory ingredients. Compressed tablets may be prepared using a binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered inhibitor moistened with an inert liquid diluent.

The tablets and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulations so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the pharmaceutical compositions of the invention include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the pharmaceutical compositions for oral administration can also include adjuvants such as wetting agents, emulsifying and suspending agents, sweetening, flavoring, coloring, perfuming, and preservative agents.

Suspensions, in addition to the pharmaceutical composition of the present invention, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

For buccal administration the pharmaceutical compositions may take the form of tablets or lozenges formulated in a conventional manner.

For administration by inhalation, the pharmaceutical compositions of the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The pharmaceutical compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions of this invention suitable for parenteral administration comprise one or more inhibitors of the invention in combination with one or more pharmaceutically acceptable sterile isotonic aqueous or non-aqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and non-aqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These pharmaceutical compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the pharmaceutical compositions. In addition, prolonged absorption of the injectable pharmaceutical form may be brought about by the inclusion of agents that delay absorption such as aluminum monostearate and/or gelatin.

In addition to the formulations described previously, the pharmaceutical compositions may also be formulated as a depot preparation. Such long acting formulations may be administered by implantation (for example subcutaneously or intramuscularly) or by intramuscular injection. Thus, for example, the pharmaceutical compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the pharmaceutical compositions of the invention are formulated into ointments, salves, gels, or creams as generally known in the art. A wash solution can be used locally to treat an injury or inflammation to accelerate healing.

In some cases, in order to prolong the therapeutic effect of an inhibitor, it is desirable to slow the absorption of the inhibitor from subcutaneous or intramuscular injection. This may be accomplished by the use of a liquid suspension of crystalline or amorphous material having poor water solubility. The rate of absorption of the inhibitor then depends upon its rate of dissolution which, in turn, may depend upon crystal size and crystalline form. Alternatively, delayed absorption of a parenterally administered inhibitor form is accomplished by dissolving or suspending the inhibitor in an oil vehicle.

Pharmaceutical compositions of the invention may be formulated for rectal or vaginal administration as a suppository, which may be prepared by mixing the compound of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active inhibitor.

Formulations of the pharmaceutical compositions of the present invention, which are suitable for vaginal administration, also include pessaries, tampons, creams, gels, pastes, foams or spray formulations containing such carriers as are known in the art to be appropriate.

Dosage forms for the topical or transdermal administration of the compound of this invention include powders, sprays, ointments, pastes, creams, lotions, gels, solutions, patches and inhalants. Such compound may be mixed under sterile conditions with a pharmaceutically acceptable carrier, and with any preservatives, buffers, or propellants which may be required.

The ointments, pastes, creams and gels may contain, in addition to the compound of the invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of the compound of the present invention to the body. Such dosage forms can be made by dissolving or dispersing an inhibitor of the present invention in the proper medium. Absorption enhancers can also be used to increase the flux of the drug across the skin. The rate of such flux can be controlled by either providing a rate controlling membrane or dispersing the compound of the present invention in a polymer matrix or gel.

Ophthalmic formulations, eye ointments, powders, solutions and the like, are also contemplated as being within the scope of this invention.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton.

A pharmaceutical composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions of the present invention can be used to provide slow or sustained release of one or more of the active ingredients using, for example, hydroxypropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

Injectable depot forms are made by forming microencapsuled matrices of the subject inhibitors in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

In a fourth aspect, the present invention relates to an HDAC10 inhibitor of the present invention, a pharmaceutically acceptable salt form of an HDAC10 inhibitor of the present invention or a pharmaceutical composition of the present invention, for use in the treatment of cancer.

In particular embodiments, autophagy is upregulated in the cells of said cancer.

In a fifth aspect, the present invention relates to a method of treating cancer, comprising the step of administering an HDAC10 inhibitor of the present invention, a pharmaceutically acceptable salt form of an HDAC10 inhibitor of the present invention, or a pharmaceutical composition of the present invention to a patient suffering from said cancer.

In particular embodiments, autophagy is upregulated in the cells of said cancer.

When the compound of the present invention are administered as pharmaceuticals, to individuals, such as humans and animals, they can be given per se or as a pharmaceutical composition containing, for example, 0.1 to 99.5% (in certain embodiments, 0.5 to 90%) of active ingredient in combination with a pharmaceutically acceptable carrier.

The present invention provides new methods of treating proliferative, degenerative and other disorders or diseases, including cancer, by administering an amount such as a therapeutically effective amount of the compound disclosed herein or a prodrug, tautomeric, pharmaceutically acceptable salt, N-oxide or stereoisomeric form thereof. The present invention further provides methods of treating proliferative, degenerative or other disorders or diseases, including cancer, by administering a therapeutically effective combination of at least the compound disclosed herein and another anti-cancer or anti-proliferative agent.

The compound of the present invention may be administered as a salt or prodrug that, upon administration to the individual, is capable of providing directly or indirectly the parent compound, such as the compound as defined herein, or that exhibits activity itself. Non-limiting examples include a pharmaceutically acceptable salt, alternatively referred to as a "physiologically acceptable salt". In addition, modifications made to the compound can affect its biological activity, in some cases increasing the activity over the parent compound. This activity can be assessed by preparing a salt or prodrug form of the compound, and testing its activity by using methods described herein or other methods known to those of skill in the art.

As will be apparent to a person skilled in the art, through the use of a prodrug of a given subject compound, an individual such as an animal administered or treated with such prodrug will be exposed to, and hence indirectly administered with, the subject compound. Such a procedure may expose those cells associated with a disease, such as a proliferative disease or disorder including cancer, to the subject compound.

All processes used to prepare the compound of the present invention and intermediates made therein are considered to be part of the present invention.

A dosage administered that will be a therapeutically effective amount of the compound sufficient, or reasonably expected by a health-care professional such as a physician, pharmacist or nurse, to result in amelioration of symptoms of, for example, the cancer or tumor will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

The subject compound may also be administered in prophylactic treatment. If the compound is administered prior to clinical manifestation of the unwanted condition (e.g., disease or other unwanted state of the host animal) then the treatment is prophylactic (i.e., it protects the individual against initiating, developing or further developing the unwanted condition). The subject compound may also be administered to prevent a condition, disorder or diseases, such as cancer, or a syndrome complex, such as heart failure or any other medical condition. This includes administration of the compound the intent of which is to reduce the frequency of, or delay the onset of, symptoms of a medical condition in an individual relative to an individual which does not receive the compound. Thus, prevention of cancer includes, for example, reducing the number of detectable cancerous growths, tumors, or malignancies in a population of patients receiving a prophylactic treatment relative to an untreated control population, delaying the appearance of detectable cancerous growths in a treated population versus an untreated control population, and/or delaying disease progression and/or improving the quality of patient life, e.g., by a statistically and/or clinically significant amount.

Toxicity and therapeutic efficacy of pharmaceutical compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit large therapeutic indices are useful for many circumstances. In certain circumstances, even therapeutic compositions that appear to exhibit debilitating or toxic side effects may be used, including circumstances where care is taken to design a delivery system that targets such therapeutic agents to the site of affected tissue in order to minimize potential damage to unaffected cells and, thereby, reduce or localize side effects.

The data obtained from cell culture assays and animal studies can be used in formulating a range of dosage for use in humans. The dosage lies preferably within a range of circulating concentrations that include the ED₅₀ with little or no toxicity. The dosage may vary within this range depending upon the dosage form employed and the route of administration utilized. For any agents used in the method of the invention, the therapeutically effective dose can be estimated initially from cell culture assays. A dose may be formulated in animal models to achieve a circulating plasma concentration range that includes the IC₅₀ (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depends upon a number of factors known to those or ordinary skill in the art, e.g., a physician. The dose(s) of the subject compound will vary, for example, depending upon the identity, size, and condition of the subject or sample being treated, further depending upon the route by which the composition is to be administered, if applicable, and the effect which the practitioner desires the therapeutic to have upon the therapeutic target of targets, such as cells, nucleic acid or polypeptides, through with the disease causes, symptoms or effects are mediated.

Exemplary doses include milligram or microgram amounts of the compound of the present invention per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram.

A person skilled in the art will appreciate that doses can also be calculated on a body surface basis. A person of 70 kg has an approximate body surface area of 1.8 square meter, and doses can be expressed as milligram or microgram amounts of the compound per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

The present invention further provides the compound as described above for therapy. In other aspects, the invention provides the compound of the present invention for prophylactic uses.

In certain embodiments, said therapy or prophylactic use is the treatment or prevention of a proliferative disorder or disease, such as a tumor or cancer. In certain embodiments, said treatment is the treatment of a cancer that can be treated by the inhibition of the activity of a protein kinase or mutant thereof, such as the inhibition of autophagy.

Thus, the present invention additionally provides a method for treating an individual, such as a mammal, having a disease-state selected from the group of proliferative disorders or diseases, or inflammatory disorders or diseases, comprising administering to said individual a therapeutically effective amount of the compound, a prodrug, or a pharmaceutical composition of the invention as described above. In certain embodiments, said individual is a human. In certain embodiments, said proliferative disorder or disease is cancer. In certain embodiments, said treatment is the treatment of a cancer that can be treated by the inhibition of the activity of a protein kinase or mutant thereof, such as the inhibition of the activity of HDAC10.

The present invention also provides a method for prophylactic treatment of an individual such as an animal, including a mammal, particularly a human, the intent of which is to reduce the frequency of, delay the onset of, or the symptoms of a medical condition, such as cancer, in a subject relative to a subject which does not receive the composition.

In a further aspect, the invention provides methods of treating or preventing an individual suffering from a disease, such as a mammal, including a domestic mammal, cat, dog, horse, sheep, cow, rodent, and human, comprising the step of exposing said individual to an amount, including a therapeutically effective amount, of the subject compound. In certain embodiments, the disease is a proliferative disorder or disease, such as a cancer or tumour. In yet another embodiment, cells associated with said proliferative disorder or disease, including tumour cells included in a cancer, are exposed to the subject compound. In certain embodiments, said compound, or a prodrug thereof, is administered to said individual. In certain embodiments, said treatment is the treatment of a cancer that can be treated by the inhibition of the activity of a protein kinase or mutant thereof, such as the inhibition of the activity of HDAC10. In certain embodiments, the disease is an inflammatory disorder or disease. In yet another embodiment, cells associated with said inflammatory disorder or disease are exposed to the subject compound. In certain embodiments, said compound, or a prodrug thereof, is administered to said individual.

In a further aspect, the invention provides a method of killing or inhibiting proliferation or growth of a cell, comprising contacting the cell with the compound of the invention. In one embodiment, the cell is cultured in-vitro, while in an alternative embodiment the cell is present in an individual. In a particular embodiment the cell is a cancer cell, for example a cell from a tumour cell line or a cell included in a tumour, including cancer cells from a tumour that can be treated by the inhibition of the activity of HDAC10.

Yet another aspect of the invention relates to the use of the compound as described above, or a prodrug thereof, for the preparation of a medicament for the treatment or prevention of a proliferative disorder or disease, including cancer, including cancers that can be treated by the inhibition of the activity of HDAC10. Additionally, the invention relates to a pharmaceutical composition comprising the compound as described above, or a prodrug thereof, and a pharmaceutically acceptable diluent, excipient or carrier, for the treatment of a proliferative disorder or disease, including cancer, including cancers that can be treated by the inhibition of the activity of a protein kinase or mutant thereof, such as the inhibition of the activity of HDAC10.

The subject compound is useful to treat various disorders or diseases, including proliferative disorders or diseases. The term "proliferative disorder or disease" is also art recognized and includes a disorder or disease affecting an individual, such as an animal, in a manner which is marked by aberrant, or otherwise unwanted, proliferation of a subset of cells of an individual. Cancer and tumors are proliferative disorders or diseases. Cells comprising or derived from a tumor will generally be understood to be a proliferating cell, typically a hyper-proliferating cell, and in other circumstances, a tumor cell may be dysplastic, or may have proliferated. In certain embodiments, said treatment is the treatment of a cancer that can be treated by the inhibition of the activity of a protein kinase or mutant thereof, such as the inhibition of the activity of HDAC10.

It will be apparent to a person skilled in the art, on reading the disclosure of the instant invention, that the methods, pharmaceutical compositions and packaged pharmaceuticals comprising the subject compound will be useful for the treatment of other proliferative disorders or diseases, or for killing or inhibiting proliferating cells including tumor cells.

the compound of the present invention may be useful in the treatment of disease processes which feature abnormal cellular proliferation, such as hyperproliferative diseases, including cancer, benign prostate hyperplasia, familial adenomatosis polyposis, neurofibromatosis, psoriasis, fungal infections, endotoxic shock, hypertrophic scar formation, inflammatory bowel disease, transplant rejection, vascular smooth muscle cell proliferation associated with atherosclerosis, psoriasis, pulmonary fibrosis, arthritis, glomerulonephritis, restenosis following angioplasty or vascular surgery, and other post-surgical stenosis and restenosis. See, for example, U.S. Patent Nos. 6,114,365 and 6,107,305.

The compound disclosed herein is expected to be useful in the therapy of proliferative or hyperproliferative disorders or diseases such as cancer, autoimmune diseases, viral diseases, fungal diseases, neurodegenerative disorders and cardiovascular disease.

In certain embodiments, tumors may be solid tumors, which are cancer of body tissues other than blood, bone marrow, or the lymphatic system. In other embodiments, tumors may be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, thyroid cancer, skin cancer, including squamous cell carcinoma, esophagus cancer, kidney cancer, bladder cancer, gall cancer, cervical cancer, ovarian cancer, lung cancer, bronchial, small and non-small-cell lung cancer, gastric, and head and neck cancer.

Hematological tumors may be leukemia, such as Acute Myelogenous Leukemia (AML), Acute Lymphoblastic Leukemia (ALL), Acute Lymphocytic Leukemia, Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin's Lymphoma, Non-Hodgkin's Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Hairy Cell Lymphoma, Extramedullary myeloma, Granulocytic Sarcomae.

Hematological tumors may also be tumors of myeloid lineage, including acute and chronic myelogenous leukemias, myelodysplastic syndrome, and promyelocytic leukaemia.

Tumors may also be of mesenchymal origin, such as fibrosarcoma and rhabdomyosarcoma. Furthermore, tumors may be tumors of the central and peripheral nervous system, such as astrocytoma, neuroblastoma, glioma, and schwannomas; and tumors may be other tumors, such as melanoma, seminoma, teratocarcinoma, osteosarcoma, xenoderoma pigmentosum, keratoctanthoma, thyroid follicular cancer, and Kaposi's sarcoma.

Tumors that are resistant or refractory to treatment with other anti-cancer or anti-proliferative agents may also benefit from treatment with the methods and pharmaceutical compositions of the present invention.

The compound disclosed herein may also be useful in the chemoprevention of cancer. Chemoprevention is defined as inhibiting the development of invasive cancer by either blocking the initiating mutagenic event or by blocking the progression of pre-malignant cells or inhibiting tumor relapse.

The compound disclosed herein may also be useful in inhibiting tumor angiogenesis and metastasis.

The compound of this invention may also be useful in combination (administered together or sequentially) with known anti-cancer treatments such as radiation therapy or with anti-cancer, anti-proliferative, cytostatic or cytotoxic agents. Other anti-cancer and anti-proliferative agents which may be used in combination with the compound of the present invention include those described herein. In combination treatment, the compound of the present invention may be further administered with any other anti-cancer and anti-proliferative agent disclosed herein.

If formulated as a fixed dose, such combination products employ the compound of this invention within the dosage range described herein and the other pharmaceutically active agent or treatment within its approved dosage range. For example, the cdc2 inhibitor olomucine has been found to act synergistically with known cytotoxic agents in inducing apoptosis (J. Cell Sci., 108, 2897 (1995)). The compound described herein may also be administered sequentially with known anti-cancer or anti-proliferative agents when a combination formulation is inappropriate. The invention is not limited in the sequence of administration; the compound described herein may be administered either prior to or after administration of the known anti-cancer or anti-proliferative agent. For example, the cytotoxic activity of the cyclin-dependent kinase inhibitor flavopiridol is affected by the sequence of administration with anticancer agents (Cancer Research, 57, 3375 (1997)).

### Further Aspects of the Invention

Another aspect the invention provides a pharmaceutical package, wherein said package includes the compound of the present invention. In certain embodiments, the package comprises instructions which indicate that said composition may be used for the treatment of an individual in need thereof, including a human. In certain other embodiments, the pharmaceutical package includes the compound of the present invention formulated together with another pharmaceutical ingredient such as an anti-cancer or anti-proliferative agent. In this case, the compound of the present invention and the other pharmaceutical ingredient may be formulated separately and in individual dosage amounts.

Other pharmaceutical ingredients that may be formulated together or separately with the compound of the present invention include but are not limited to other anti-cancer and anti-proliferative agents such as described above. In certain still further embodiments, the pharmaceutical package comprises instructions to treat a patient in need of such treatment. In yet another aspect the invention provides a pharmaceutical package for treating an individual suffering from a proliferative disorder or disease, such as a tumor or a cancer, wherein said package includes at least the compound of the present invention. In certain still further embodiments, the pharmaceutical package comprises instructions to treat the disorder.

As used herein the term "pharmaceutical package" or "pharmaceutical pack" refer to any packaging system for storing and dispensing individual doses of medication. Preferably the pharmaceutical package contains sufficient daily dosage units appropriate to the treatment period or in amounts which facilitate the patient's compliance with the regimen. In certain embodiments, the pharmaceutical pack comprises one or more vessels that include the active ingredient, e.g., the compound of the present invention. Such vessel can be a container such as a bottle, vial, syringe, or capsule, or may be a unit dosage form such as a pill. The active ingredient may be provided in the vessel in a pharmaceutically acceptable form or may be provided, for example, as a lyophilized powder. In further embodiments, the pharmaceutical pack may further include a solvent to prepare the active ingredient for administration. In certain embodiments, the active ingredient may be already provided in a delivery device, such as a syringe, or a suitable delivery device may be included in the pack. The pharmaceutical package may comprise pills, liquids, gels, tablets, dragees or the pharmaceutical preparation in any other suitable form. The package may contain any number of daily pharmaceutical dosage units. The package may be of any shape, and the unit dosage forms may be arranged in any pattern, such as circular, triangular, trapezoid, hexagonal or other patterns. One or more of the doses or subunits may be indicated, for example to aid the doctor, pharmacist or patient, by identifying such dose or subunits, such as by employing color-coding, labels, printing, embossing, scorings or patterns. The pharmaceutical package may also comprise instructions for the patient, the doctor, the pharmacist or any other related person.

Some embodiments comprise the administration of more than one active ingredient, including the compound as disclosed herein. Such administration may occur concurrently or sequentially. The active ingredients may be formulated together such that one administration delivers both components. Alternatively the active ingredients may be formulated separately. The pharmaceutical package may comprise the compound of the present invention and the other pharmaceutical ingredient in a single formulation, i.e., they are formulated together, or the compound of the present invention and the other pharmaceutical ingredient in individual formulations, i.e., they are formulated separately. Each formulation may comprise the compound of the present invention and the other pharmaceutical ingredient in individual dosage amounts (in approximately equal or unequal amounts). Administration of the compound of the present invention and the other pharmaceutical ingredient results in a concentration that results in a therapeutically effective amount of the combination.

As used herein, the term "instructions" means a product label and/or documents or other information describing relevant materials or methodologies pertaining to assembly, preparation or use of a kit or packaged pharmaceutical. These materials may include any combination of the following: background information, steps or procedures to follow, list of components, proposed dosages, warnings regarding possible side effects, instructions for administering the drug, technical support, and any other related documents. Instructions can be supplied in printed form, such as a package label or a package insert. Instructions for a packaged pharmaceutical or a pharmaceutical composition can be inserted in a delivery carton or finished package, e.g., as a package insert, and the text of such has been approved by a competent regulatory authority such as the Food and Drug Administration (FDA) of the United States. Alternatively or complementarily, instruction may also be stored in electronic form, e.g., on a computer-readable storage medium such as a computer-readable memory device, a centralized database, magnetic media such as hard disks, floppy disks, and magnetic tape; optical media such as compact discs, CD-ROMs and holographic devices; magneto-optical media such as floptical disks; and hardware devices that are specially configured to store and execute program code, such as application-specific integrated circuits (ASICs), programmable logic devices (PLDs) and ROM (read only memory) and RAM (random access memory) devices. Instructions may comprise a web address of an internet website from which more detailed instructions may be downloaded, or a recorded presentation. Instructions can contain one or multiple documents or future updates.

Another aspect of the invention provides the use of the compound of Formula (I) to inhibit activity of HDAC10. In another embodiment of this aspect, the compound of Formula (I) is used for the preparation of a composition for the inhibition of activity of HDAC10. In yet another embodiment, the invention provides methods to inhibit HDAC10.

Thus, in one aspect the invention relates to a pharmaceutical composition, including the compound of the present invention, and a pharmaceutically acceptable diluent, excipient or carrier.

In certain embodiments, such pharmaceutical composition comprises a therapeutically effective amount of said compound or prodrug.

In certain embodiments, such pharmaceutical composition is for the treatment of an individual in need thereof.

In certain embodiments of such pharmaceutical composition, said individual is a human.

In another aspect the invention relates to a pharmaceutical package, including a pharmaceutical composition of the present invention, and instructions which indicate that said pharmaceutical composition may be used for the treatment of an individual in need thereof.

In certain embodiments of such pharmaceutical package, said instructions indicate that said pharmaceutical composition may be used for the treatment of a human.

In certain embodiments of such pharmaceutical package, said instructions indicate that said pharmaceutical composition may be used for the treatment of an individual suffering from a proliferative disorder or disease.

In certain such embodiments, said individual is a human.

In another aspect the invention relates to a method for treating a proliferative disorder or disease in an individual, comprising administering a therapeutically effective amount of the compound or a pharmaceutical composition of the present invention.

In certain embodiments of such method, said individual is a mammal selected from: domestic mammal, cat, dog, horse, sheep, cow, rodent, and human.

In certain embodiments of such method, said mammal is a human.

In another aspect the invention relates to a method for treating a proliferative disorder or disease in an individual, comprising exposing cells included in said disorder or disease to the compound of the present invention.

In certain embodiments of such method, said compound, or a prodrug thereof, is administered to said individual.

In certain embodiments of such method, said individual is a mammal selected from: domestic mammal, cat, dog, horse, sheep, cow, rodent, and human.

In certain embodiments of such method, said mammal is a human.

In another aspect the invention relates to a method for inhibiting cell proliferation, comprising contacting a cell with the compound of the present invention.

In another aspect the invention relates to the compound of the present invention for the preparation of a medicament for the treatment of a proliferative disorder or disease.

In another aspect the invention relates to a pharmaceutical composition comprising the compound of the present invention and a pharmaceutically acceptable carrier, diluent or excipient, for the treatment of a proliferative disorder or disease.

In certain embodiments of a pharmaceutical package, method, use or pharmaceutical composition of the present invention, the proliferative disorder or disease is a cancer.

### EXAMPLES

### Experimental Section:

Chemicals and solvents were purchased from commercial sources at the highest level of purity and used without purification. Anhydrous tetrahydrofuran was prepared with an MBraun SPS800 Solvent Purification System. Thin layer chromatography (TLC) was carried out on glass silica plates (TLC Silica gel 60 F₂₅₄; Merck). TLC visualization was accomplished using 254 and 366 nm UV light or ninhydrin stain.

Analytical HPLC/MS was performed on an Agilent 1260 Infinity system.

**Analytical HPLC-Methods:**

| method name | column | solvent system | gradient |
|---|---|---|---|
| acidic A | Kinetex 2.6 µm C18 100 Å, LC column 50 x 2.1 mm; 40 °C; flow rate: 0.06 mL/min | A: H₂O, 0.01% HCOOH; B: MeCN, 0.01% HCOOH | 1% B 90% B over 6 min, then 90% B to 99% B over 2 min |

| | | | |
|---|---|---|---|
| UV-detection at 254 nm | | | |

Preparative HPLC was performed on an Agilent 1260 Infinity system.

**Preparative HPLC Methods:**

| method name | column | solvent system | gradient |
|---|---|---|---|
| acidic A | Kinetex 5µm C18 100 Å, AXIA packed column 250 x 21.2 mm; 20°C; flow rate: 15.0 mL/min | A: H₂O, 0.05% TFA; B: MeCN, 0.05% TFA | 1 → 10% B in 3min, 10 → 45% B in 11 min |
| | | | 100% B for 4 min |
| basic A | Gemini 5µm C18 110 Å, AXIA packed column 250 x 21.2 mm; 20°C; flow rate: 15.0 mL/min | A: H₂O, 0.1% NH₃; B: MeCN | 1 → 40% B in 13min, |
| | | | 100% B for 6 min |

| | | | |
|---|---|---|---|
| UV-detection at 254 and 230 nm | | | |

Solvent was removed by lyophilisation with a Christ alpha 2-4 LD plus freeze-dyer.

### General procedure for preparation of hydroxamic acids

To a stirring solution of methyl ester (typically 50-200 mg, 1.0 eq) in 1,4-dioxane (0.5-2 mL), an aqueous solution of hydroxylamine (50%, 0.5-2 mL, min. 30 eq) was added together with potassium cyanide (0.6 eq). The reaction was stirred for 24 hours at room temperature. The reaction mixture was concentrated *in vacuo,* then dissolved in water/acetonitrile and filtered. Purification was carried out by preparative HPLC. Product fractions were combined and solvent was removed by lyophilization.

### Synthesis of DKFZ-621 (N-hydroxy-4-((3-oxo-3-(phenylamino)propyl)amino)butanamide):

Methyl 4-aminobutanoate hydrochloride (1.0167 g, 6.62 mmol, 1.0 eq) was added to a suspension of N-phenylprop-2-enamide (979.9 mg, 6.66 mmol, 1.0 eq) in EtOH (80 ml). Potassium carbonate (930.5 mg, 6.73 mmol, 1.0 eq) was added and the cloudy mixture was stirred for 24 h at 20°C. The reaction mixture was poured into water (200 ml). The formed oily substance was extracted with CHCl₃ (2x35 ml), the extract was washed with water, dried over MgSO₄, and the solvent was evaporated. The crude product was purified by flash column chromatography (CH₂Cl₂:MeOH, 3% to CH₂Cl₂:MeOH, 10% + 0.5% NH₄OH) affording methyl 4-((3-oxo-3-(phenylamino)propyl)amino)butanoate in a yield of 24% (414.3 mg, 1.60 mmol) as a yellow oil. The title compound was prepared from methyl 4-((3-oxo-3-(phenylamino)propyl)amino)butanoate (393.1 mg, 1.49 mmol) according to the general procedure for preparation of hydroxamic acids and purified by HPLC using acidic method A, affording the trifluoroacetate salt of the title compound as a colorless oil in a yield of 3% (14.6 mg, 0.045 mmol).
**¹H NMR** (400 MHz, DMSO-*d*₆) δ 10.52 (s, 1H), 10.21 (s, 1H), 8.63 (s, 2H), 7.60-7.58 (m, 2H), 7.31 (t, J = 7.7 Hz, 2H), 7.05 (t, J = 7.3 Hz, 1H), 3.23-3.20 (m, 2H), 2.98-2.92 (m, 2H), 2.76 (t, J = 6.8 Hz, 2H), 2.07 (t, J = 7.4 Hz, 2H), 1.83 (qi, J = 7.1 Hz, 2H) ppm.
**¹³C NMR** (101 MHz, DMSO-*d*₆) δ 168.2, 168.1, 138.9, 128.8, 123.5, 119.2, 46.7, 42.7, 32.2, 29.2, 21.6 ppm. TFA signals not listed
**MS** *m*/*z:* [M+H]⁺: 266.2, [M-H]⁻: 264.2

### Synthesis of DKFZ-711 (4-(methyl(3-oxo-3-(phenylamino)propyl)amino)-N-hydroxybutanamide):

*N*-phenylacrylamide (2.00 g, 13.59 mmol, 1.0 eq), *N*-Me-gamma-aminobutyric acid·HCl (2.30 mg, 14.95 mmol, 1.1 eq) and potassium carbonate (3.76 g, 27.18 mmol, 2.0 eq) were suspended in water/ethanol (1:1, 40 mL). The reaction mixture was stirred at 70 °C for 7 h, then cooled to RT, acidified to pH ∼1 with 3 M HCI and evaporated to dryness. The residue was suspended in methanol and stirred at RT for 3 days, then concentrated *in vacuo.* The product was purified by column chromatography (MeOH/DCM, 10:90) to give 3.492 g (82%) of methyl 4-(methyl(3-oxo-3-(phenylamino)propyl)amino)butanoate hydrochloride as a yellow amorphous solid (**MS** *m*/*z*: [M+H]⁺: 251.1 [M-H]⁻: 249.1). A solution of methyl 4-(methyl(3-oxo-3-(phenylamino)propyl)amino)butanoate hydrochloride (250 mg, 0.794 mmol, 1.0 eq) in water (20 ml) was basified with a saturated NaHCO₃ solution until pH 10.0. The mixture was extracted with dichloromethane (3 x 20 ml), the combined organic phases were dried over magnesium sulfate, filtered and solvent was removed *in vacuo* yielding quantitatively the free-base. To a solution of potassium cyanide (31.5 mg, 0.484 mmol, 0.61 eq) in 1,4-dioxane (1.70 mL), were added an aqueous solution of hydroxylamine (50%, 1.70 mL, 25.4 mmol, 32 eq) and all of the free-base. The reaction was stirred for 24 hours at RT. The reaction was stopped with the removal of the excess of reagent and solvent *in vacuo,* then crude product was dissolved in water (2 mL) with addition of a minimal amount of acetonitrile. Purification was carried out by RP-HPLC using method acidic A. Solvent was removed by lyophilization, affording the trifluoroacetate salt of the title compound as a hygroscopic resin, in a yield of 62% (193 mg, 0.491 mmol).
**R_{F}** 0.21 (MeOH/NH₄OH/CH₂Cl₂ 10/0.5/89.5)
**¹H NMR** (400 MHz, D₂O) δ 7.48 - 7.41 (m, 4H), 7.28 (ddt, *J* = 8.6, 5.5, 3.1 Hz, 1H), 3.72 - 3.40 (m, 2H), 3.35 - 3.15 (m, 2H), 3.02 - 2.96 (m, 2H), 2.96 - 2.90 (m, 3H), 2.32 (t, *J* = 7.2 Hz, 2H), 2.08 (p, *J* = 7.2 Hz, 2H) ppm.
**¹³C NMR** (101 MHz, D₂O) δ 174.0, 173.0, 139.3, 132.1, 128.6, 124.7, 58.4, 54.7, 42.9, 32.9, 31.8, 22.5 ppm. TFA signals not listed.
**HR-MS** *m*/*z* calcd. for C₁₄H₂₂N₃O₃⁺ [M+H⁺]: 280.1659; found 280.1656.

### Synthesis of 4-((3-oxo-3-(phenylamino)propyl)amino)butanoic acid hydrochloride:

To a stirred solution of gamma-aminobutyric acid (3.14 g, 30.45 mmol, 2.2 eq) and potassium carbonate (2.68 g, 19.40 mmol 1.4 eq) in an ethanol (17 mL)/water (27 mL) mixture heated to 70 °C, was added dropwise a solution of *N*-phenylacrylamide (2.00 g, 13.59 mmol, 1.0 eq) in ethanol (10 mL). The reaction was stirred at 70 °C for 3.5 h, cooled to RT and extracted with CH₂Cl₂. Organic layers were discarded and the aqueous phase was acidified with HCI, then evaporated to dryness to yield a white solid. The residue was dissolved in a water/methanol mixture at 50 °C and crystallized by concentrating somewhat under reduced pressure and then slowly cooling to 0 °C. Crystals were filtered and washed once with ice cold water, then with acetone, affording the title compound as white crystals in a yield of 32% (1.239 g, 4.32 mmol).
**TLC** *R*_{f} 0.05 (H₂O/MeCN 10:90)
**¹H NMR** (400 MHz, MeOD) δ 7.62 - 7.56 (m, 2H), 7.36 - 7.27 (m, 2H), 7.14 - 7.08 (m, 1H), 3.36 (t, *J* = 6.5 Hz, 2H), 3.17 - 3.11 (m, 2H), 2.88 (t, *J* = 6.4 Hz, 2H), 2.49 (t, *J* = 7.0 Hz, 2H), 2.01 (p, *J* = 7.0 Hz, 2H) ppm.
**¹³C NMR** (101 MHz, MeOD) δ 176.0, 170.1, 139.6, 129.8, 125.3, 121.1, 48.4, 44.8, 32.8, 31.6, 22.4 ppm.
**HPLC/MS** [M+H]⁺: 251.1 [M-H]⁻: 249.1

### Synthesis of DKFZ-714 (4-(ethyl(3-oxo-3-(phenylamino)propyl)amino)-N-hydroxybutanamide):

4-((3-Oxo-3-(phenylamino)propyl)amino)butanoic acid hydrochloride (100 mg, 0.349 mmol, 1.0 eq) and sodium triacetoxyborohydride (111 mg, 0.523 mmol, 1.5 eq) were dissolved in dry THF (1.0 mL) under an atmosphere of nitrogen at 0 °C. Ice-cold acetaldehyde (154 µL, 3.49 mmol, 10 eq), then further dry THF (0.5 mL) and finally, triethylamine (70.6 µL, 0.697 mmol, 2.0 eq) were added. Upon complete addition the cooling bath was removed and the reaction mixture was stirred for 2 h at room temperature. The mixture was diluted with water (10 mL), basified with a saturated NaHCO₃ solution until pH 12.0, then the solvent was removed *in vacuo.* The resulting pale yellow powder was dissolved in water (10 mL), solution was acidified with hydrochloric acid (25% in water) in a dropwise manner until pH 1.0, then the solvent was removed *in vacuo.* Methanol (25 mL) was added to the resulting powder and esterification was done by refluxing for 2 h, then stirring overnight at room temperature, after which the solvent was removed *in vacuo.* The resulting powder was dissolved in water (20 mL), the clear solution became cloudy upon basification with a saturated NaHCO₃ solution until pH 10.0. The mixture was extracted with dichloromethane (3 x 20 ml), the combined organic phases were dried over magnesium sulfate, filtered and solvent was removed *in vacuo.* The hydroxamic acid was prepared from the methyl ester (70.0 mg, 0.239 mmol, 1.0 eq) according to the general procedure and purified by HPLC using acidic method A, affording the trifluoroacetate salt of the title compound as a yellow hygroscopic resin in a yield of 53% (51.8 mg, 0.127 mmol).
**R_{F}** 0.082 (MeOH/CH₂Cl₂ 10/90)
**¹H NMR** (400 MHz, D₂O) δ 7.48 - 7.41 (m, 4H), 7.32 - 7.22 (m, 1H), 3.59 - 3.50 (m, 2H), 3.31 (q, *J* = 7.3 Hz, 2H), 3.28 - 3.18 (m, 2H), 2.95 (t, *J* = 6.8 Hz, 2H), 2.36 - 2.28 (m, 2H), 2.12 - 2.01 (m, 2H), 1.34 (t, *J* = 7.3 Hz, 3H) ppm.
**¹³C NMR** (101 MHz, D₂O) δ 174.0, 173.0, 139.3, 132.1, 128.6, 124.7, 54.7, 51.3, 51.1, 32.9, 31.9, 22.1, 11.0 ppm. TFA signals not listed.
**HR-MS** *m*/*z* calcd. for C₁₅H₂₄N₃O₃⁺ [M+H⁺]: 294.1812; found: 294.1816.

### Synthesis of DKFZ-715 (4-(isopropyl(3-oxo-3-(phenylamino)propyl)amino)-N-hydroxybutanamide):

4-((3-Oxo-3-(phenylamino)propyl)amino)butanoic acid hydrochloride (150 mg, 0.523 mmol, 1.0 eq) and sodium triacetoxyborohydride (166 mg, 0.785 mmol, 1.5 eq) were dissolved in dry THF (1.0 mL) under an atmosphere of nitrogen. Acetone (390 µL, 5.23 mmol, 10 eq), then further dry THF (1.0 mL) and finally, triethylamine (150 µL, 1.05 mmol, 2.0 eq) were added. The reaction mixture was stirred for 3 h at room temperature. The mixture was diluted with water (10 mL), basified with a saturated NaHCO₃ solution until pH 12.0, then the solvent was removed *in vacuo.* The resulting pale yellow powder was dissolved in water (10 mL), solution was acidified with hydrochloric acid (25% in water) in a dropwise manner until pH 1.0, then the solvent was removed *in vacuo.* Methanol (25 mL) was added to the resulting powder and esterification was done by refluxing for 2 h, then addition of dry hydrochloric acid (2 M in diethyl ether 0.52 mL, 1.05 mmol, 2 eq,) and stirring overnight at room temperature, after which the solvent was removed *in vacuo.* The resulting powder was dissolved in water (20 mL), the clear solution became cloudy upon basification with a saturated NaHCO₃ solution until pH 10.0. The mixture was extracted with dichloromethane (3 x 20 ml), the combined organic phases were dried over magnesium sulfate, filtered and solvent was removed *in vacuo* to give 144 mg of crude ester. The hydroxamic acid was prepared from the methyl ester (89.0 mg, 0.290 mmol, 1.0 eq) according to the general procedure and purified by HPLC using acidic method A, affording the trifluoroacetate salt of the title compound as a yellow hygroscopic resin in a yield of 27% (33.0 mg, 0.078 mmol).
**TLC** *R*_{f} 0.042 (MeOH/CH₂Cl₂ 10/90)
**¹H NMR** (400 MHz, D₂O) δ 7.48 - 7.39 (m, 4H), 7.28 (dtd, *J* = 8.4, 5.0, 3.1 Hz, 1H), 3.78 (hept, *J* = 6.6 Hz, 1H), 3.59 (dt, *J* = 13.9, 7.0 Hz, 1H), 3.39 (dt, *J* = 13.4, 6.5 Hz, 1H), 3.25 (ddd, *J* = 13.3, 9.0, 6.8 Hz, 1H), 3.14 (ddd, *J* = 13.4, 9.0, 6.5 Hz, 1H), 2.94 (t, *J* = 6.8 Hz, 2H), 2.33 (t, *J* = 7.0 Hz, 2H), 2.14 - 1.96 (m, 2H), 1.35 (t, *J* = 7.1 Hz, 6H) ppm.
**¹³C NMR** (101 MHz, D₂O) δ 174.0, 173.1, 139.3, 132.1, 128.6, 124.7, 58.6, 52.7, 48.9, 33.5, 32.1, 23.2, 18.7, 18.3 ppm. TFA signals not listed.
**HR-MS** m/z calcd. for C₁₆H₂₆N₃O₃⁺ [M+H⁺]: 308.1969, found: 308.1974

### Synthesis of DKFZ-716 (4-(propyl(3-oxo-3-(phenylamino)propyl)amino)-N-hydroxybutanamide):

4-((3-Oxo-3-(phenylamino)propyl)amino)butanoic acid (150 mg, 0.523 mmol, 1.0 eq) and sodium triacetoxyborohydride (166 mg, 0.785 mmol, 1.5 eq) were dissolved in dry THF (1.0 mL) under an atmosphere of nitrogen at 0 °C. Ice-cold propanal (380 µL, 5.23 mmol, 10 eq), then further dry THF (0.5 mL) and finally, triethylamine (150 µL, 1.05 mmol, 2.0 eq) were added. Upon complete addition the cooling bath was removed and the reaction mixture was stirred for 2 h at room temperature. The mixture was diluted with water (10 mL), basified with a saturated NaHCO₃ solution until pH 12.0, then the solvent was removed *in vacuo.* The resulting pale yellow powder was dissolved in water (10 mL), solution was acidified with hydrochloric acid (25% in water) in a dropwise manner until pH 1.0, then the solvent was removed *in vacuo.* Methanol (25 mL) was added to the resulting powder and esterification was done by refluxing for 2 h, then stirring overnight at room temperature, after which the solvent was removed *in vacuo.* The resulting powder was dissolved in water (20 mL), the clear solution became cloudy upon basification with a saturated NaHCO₃ solution until pH 10.0. The mixture was extracted with dichloromethane (3 x 20 ml), the combined organic phases were dried over magnesium sulfate, filtered and solvent was removed *in vacuo.* The hydroxamic acid was prepared from the methyl ester (127 mg, 0.396 mmol, 1.0 eq) according to the general procedure and purified by HPLC using acidic method A, affording the trifluoroacetate salt of the title compound as a yellow hygroscopic resin in a of yield 66% (110 mg, 0.260 mmol).
**TLC** *R*_{f} 0.17 (MeOH/CH₂Cl₂ 10/90)
**¹H NMR** (400 MHz, D₂O) δ 7.48 - 7.39 (m, 4H), 7.27 (tt, *J* = 5.6, 2.9 Hz, 1H), 3.59 - 3.48 (m, 2H), 3.20 (dt, *J* = 19.4, 9.8 Hz, 4H), 2.93 (d, *J* = 6.5 Hz, 2H), 2.30 (ddd, *J* = 7.4, 5.2, 2.0 Hz, 2H), 2.10 - 1.97 (m, 2H), 1.83 - 1.67 (m, 2H), 0.98 (td, *J* = 7.4, 1.6 Hz, 3H) ppm.
**¹³C-DEPT NMR** (101 MHz, D₂O) δ 129.3 (CH), 125.8 (CH), 121.8 (CH), 55.0 (CH₂), 52.4 (CH₂), 48.8 (CH₂), 30.0 (CH₂), 29.0 (CH₂), 19.2 (CH₂), 16.9 (CH₂), 10.1 (CH₃) ppm.
**HR-MS** m/z calcd. for C₁₆H₂₆N₃O₃⁺ [M+H⁺]: 308.1969, found: 308.1974

### Synthesis of DKFZ-717 (4-(butyl(3-oxo-3-(phenylamino)propyl)amino)-N-hydroxybutanamide):

4-((3-Oxo-3-(phenylamino)propyl)amino)butanoic acid (150 mg, 0.523 mmol, 1.0 eq) and sodium triacetoxyborohydride (166 mg, 0.785 mmol, 1.5 eq) were dissolved in dry THF (1.0 mL) under an atmosphere of nitrogen at 0 °C. Ice-cold butanal (470 µL, 5.23 mmol, 10 eq), then further dry THF (0.9 mL) and finally, triethylamine (150 µL, 1.05 mmol, 2.0 eq) were added. Upon complete addition the cooling bath was removed and the reaction mixture was stirred for 2 h at room temperature. The mixture was diluted with water (10 mL), basified with a saturated NaHCO₃ solution until pH 12.0, then the solvent was removed *in vacuo.* The resulting pale yellow powder was dissolved in water (10 mL), solution was acidified with hydrochloric acid (25% in water) in a dropwise manner until pH 1.0, then the solvent was removed *in vacuo.* Methanol (25 mL) was added to the resulting powder and esterification was done by refluxing for 2 h, then stirring overnight at room temperature, after which the solvent was removed *in vacuo.* The resulting powder was dissolved in water (20 mL), the clear solution became cloudy upon basification with a saturated NaHCO₃ solution until pH 10.0. The mixture was extracted with dichloromethane (3 x 20 ml), the combined organic phases were dried over magnesium sulfate, filtered and solvent was removed *in vacuo.* The hydroxamic acid was prepared from the methyl ester (127 mg, 0.396 mmol, 1.0 eq) according to the general procedure and purified by HPLC using acidic method A, affording the trifluoroacetate salt of the title compound as a yellow hygroscopic resin in a yield of 74% (127 mg, 0.292 mmol).
**TLC** *R*_{f} 0.083 (MeOH/CH₂Cl₂ 10/90)
**¹H NMR** (400 MHz, D₂O) δ 7.46 - 7.40 (m, 4H), 7.30 - 7.22 (m, 1H), 3.59 - 3.48 (m, 2H), 3.27 - 3.14 (m, 4H), 2.98 - 2.88 (m, 2H), 2.35 - 2.25 (m, 2H), 2.10 - 1.98 (m, 2H), 1.77 - 1.65 (m, 2H), 1.38 (p, *J* = 7.5 Hz, 2H), 0.97 - 0.89 (m, 3H) ppm.
**¹³C NMR** (101 MHz, D₂O) δ 171.2, 170.2, 136.5, 129.3, 125.8, 121.9, 53.3, 52.4, 48.9, 30.0, 29.0, 25.2, 19.3 (2 CH₂), 12.8 ppm. TFA signals not listed.
**HR-MS** m/z calcd. for C₁₇H₂₈N₃O₃⁺ [M+H⁺]: 322.2125, found: 322.2127

### Synthesis of DKFZ-718 (4-(benzyl(3-oxo-3-(phenylamino)propyl)amino)-N-hydroxybutanamide):

4-((3-Oxo-3-(phenylamino)propyl)amino)butanoic acid hydrochloride (150 mg, 0.523 mmol, 1.0 eq) and sodium triacetoxyborohydride (166 mg, 0.785 mmol, 1.5 eq) were dissolved in dry THF (1.0 mL) under an atmosphere of nitrogen at 0 °C. Benzaldehyde (540 µL, 5.23 mmol, 10 eq), then further dry THF (1.0 mL) and finally, triethylamine (150 µL, 1.05 mmol, 2.0 eq) were added. Upon complete addition the cooling bath was removed and the reaction mixture was stirred for 2 h at room temperature. The mixture was diluted with water (10 mL), basified with a saturated NaHCO₃ solution until pH 12.0, then the solvent was removed *in vacuo.* The resulting pale yellow powder was dissolved in water (10 mL), solution was acidified with hydrochloric acid (25% in water) in a dropwise manner until pH 1.0, then the solvent was removed *in vacuo.* Methanol (25 mL) was added to the resulting powder and esterification was done by refluxing for 2 h, then stirring overnight at room temperature, after which the solvent was removed *in vacuo.* The resulting powder was dissolved in water (20 mL), the clear solution became cloudy upon basification with a saturated NaHCO₃ solution until pH 10.0. The mixture was extracted with dichloromethane (3 x 20 ml), the combined organic phases were dried over magnesium sulfate, filtered and solvent was removed *in vacuo.* The hydroxamic acid was prepared from the methyl ester (115 mg, 0.324 mmol, 1.0 eq) according to the general procedure and purified by HPLC using acidic method A, affording the trifluoroacetate salt of the title compound as an off-white solid in a yield of 78% (119 mg, 0.254 mmol).
**TLC** *R*_{f} 0.17 (MeOH/CH₂Cl₂ 10/90)
**¹H NMR** (400 MHz, MeOD) δ 7.62 - 7.48 (m, 7H), 7.35 - 7.29 (m, 2H), 7.11 (tt, *J* = 7.4, 1.2 Hz, 1H), 4.45 (s, 2H), 3.54 (t, *J* = 6.8 Hz, 2H), 3.28 (t, *J* = 7.4 Hz, 2H), 2.91 (t, *J* = 6.8 Hz, 2H), 2.27 (t, *J* = 6.3 Hz, 2H), 2.09 (p, *J* = 6.7 Hz, 2H) ppm.
**¹³C NMR** (101 MHz, MeOD) δ 171.4, 170.1, 139.4, 132.1, 131.3, 130.8, 130.6, 129.9, 125.5, 121.3, 58.9, 54.6, 50.3, 31.1, 30.6, 20.7 ppm. TFA signals not listed.
**HR-MS** *m*/*z* calcd. for C₂₀H₂₆N₃O₃⁺ [M+H⁺]: 356.1969, found: 356.1972

### Synthesis of DKFZ-724 (4-(cyclopropyl(3-oxo-3-(phenylamino)propyl)amino)-N-hydroxybutanamide):

To a Schlenk tube charged with 4-((3-oxo-3-(phenylamino)propyl)amino)butanoic acid hydrochloride (150 mg, 0.523 mmol, 1.0 eq) and sodium cyanoborohydride (49.3 mg, 0.785 mmol, 1.5 eq), 1.5 mL of degassed water, (1-ethoxycyclopropoxy)trimethylsilane (1.05 mL, 5.23 mmol, 10 eq) and 37% HCI (51.5 µL, 0.523 mmol, 1.0 eq) were added under argon atmosphere. The mixture was stirred vigorously for 7 d and more sodium cyanoborohydride (2.2 eq) was added in small portions as a solution in degassed water until complete consumption of starting material. The reaction was stopped by evaporating to dryness, the residue was resuspended in methanol (15 mL), acidified with 2 M HCI in Et₂O, and stirred overnight at room temperature, after which the solvent was removed *in vacuo.* The resulting powder was dissolved in water (20 mL), the clear solution became cloudy upon basification with a saturated NaHCO₃ solution until pH 10.0. The mixture was extracted with dichloromethane (3 x 20 ml), the combined organic phases were dried over magnesium sulfate, filtered and solvent was removed *in vacuo.* The hydroxamic acid was prepared from the methyl ester (115 mg, 0.378 mmol, 1.0 eq) according to the general procedure and purified by HPLC using acidic method A, affording the trifluoroacetate salt of the title compound as a white solid in a yield of 21% (32.8 mg, 0.078 mmol).
**TLC** *R*_{f} 0.17 (MeOH /CH₂Cl₂ 10/90)
**¹H NMR** (400 MHz, D₂O) δ 7.50 - 7.40 (m, 4H), 7.29 (qt, *J* = 5.5, 2.9 Hz, 1H), 3.71 (t, *J* = 6.7 Hz, 2H), 3.42 - 3.31 (m, 2H), 3.04 (t, *J* = 6.7 Hz, 2H), 2.86 (ddd, *J* = 11.2, 7.2, 4.6 Hz, 1H), 2.33 (t, *J* = 7.1 Hz, 2H), 2.15 (dq, *J* = 14.6, 7.2 Hz, 2H), 1.06 (dd, *J* = 13.8, 3.2 Hz, 2H), 1.05 (s, 2H) ppm.
**¹³C NMR** (101 MHz, D₂O) δ 174.1, 173.1, 139.3, 132.1, 128.6, 124.7, 58.1, 54.0, 40.4, 33.3, 32.0, 22.3, 7.3 (2 CH₂) ppm. TFA signals not listed.
**MS** m/z: [M+H]⁺: 306.2, [M-H]⁻: 304.2

### Synthesis of DKFZ-728 (N-(2-((4-(hydroxyamino)-4-oxobutyl)(methyl)amino)ethyl)benzamide):

To a stirring solution of *N*-methyl-γ-aminobutyric acid·HCl (237 mg, 1.55 mmol, 1.0 eq), 2 M aqueous NaOH (2.5 mL, 5.0 mmol, 3.2 eq) and a solution of Boc-2-chloroethanamine (278 mg, 1.55 mmol, 1.0 eq) in 2.5 mL MeOH was added. The mixture was refluxed for 24 h, then the mixture was cooled to RT, acidified with HCI to pH ∼2 and 2 M solution of TMSCHN₂ in hexane (2.15 mL, 4.34 mmol, 2.8 eq) was added slowly at RT until yellow color persisted. Reaction was quenched with 2 mL of AcOH, then basified with K₂CO₃, extracted with DCM, org. phases were dried over MgSO₄, filtered and concentrated in *vacuo,* which yielded 163 mg of crude product. Flash column chromatography with 20 g silica using MeOH/DCM, 10:90 + 0,5% NH₄OH as mobile phase gave 73.5 mg (17% over 2 steps) of Methyl 4-((2-((tert-butoxycarbonyl)amino)ethyl)(methyl)amino)butanoate as pale yellow oil. (*m*/*z:* [M+H]⁺: 275.2). A solution of this compound (51.3 mg, 0.133 mmol, 1.0 eq) in 5 mL DCM/TFA (4:1) was stirred for 30 min at RT, then concentrated in *vacuo.* The yellow residue was dissolved in 5 mL DCM and 3 mL saturated aqueous NaHCO₃ solution was added. Under vigorous stirring, benzoyl chloride (18.4 µL, 0.160 mmol, 1.2 eq) was added at RT. The mixture was extracted with DCM, org. phases were dried over MgSO₄, filtered and concentrated in *vacuo,* yielding 32.3 mg of methyl 4-((2-benzamidoethyl)(methyl)amino)butanoate (m/z: [M+H]⁺: 279.2). The title compound was prepared from methyl 4-((2-benzamidoethyl)(methyl)amino)butanoate (92 mg, 0.322 mmol, 1.0 eq) according to the general procedure for preparation of hydroxamic acids and purified by HPLC using basic method A, affording an off-white solid in a yield of 32% (28.8 mg, 0.254 mmol).
**TLC** *R*_{f} 0.26 (MeOH/CH₂Cl₂, 20:80)
**¹H NMR** (600 MHz, DMSO-*d*₆) δ 10.34 (s, 1H), 8.68 (s, 1H), 8.38 (t, *J* = 5.7 Hz, 1H), 7.84 - 7.80 (m, 2H), 7.53 - 7.49 (m, 1H), 7.47 - 7.43 (m, 2H), 3.39 - 3.31 (m, overlapped with water peak), 2.47 (t, *J* = 7.0 Hz, 2H), 2.32 (t, *J* = 7.2 Hz, 2H), 2.19 (s, 3H), 1.96 (t, *J* = 7.4 Hz, 2H), 1.62 (p, *J* = 7.3 Hz, 2H) ppm.
**¹³C NMR** (151 MHz, DMSO-*d*₆) δ 22.9, 30.1, 37.2, 41.9, 56.1, 56.5, 127.1, 128.3, 131.0, 134.6, 166.1, 169.1 ppm.
**MS** *m*/*z:* [M+H]⁺: 280.2, [M-H]⁻: 278.2

### Biological Testing

### HDAC6/HDAC10 assay:

HDAC6 and HDAC10 binding data were measured using a BRET assay as described in reference 41. In detail, it was performed as described by the kit manufacturer in a 96-well plate (3600, Corning) format with 1.9x10⁴ cells per well and a tracer concentration of 0.3 µM. Inhibitors were tested at ten 1:4 serial dilutions in triplicates ranging from 129 pM to 40 µM. Drug dosing was performed from 10 mM and 1 mM DMSO stock solutions with a D300e Digital Dispenser (Tecan), DMSO concentrations were normalized to 0.5% for all wells. Note: Due to the dosing increments of the drug printer, the dilution factor is not entirely stable over all dose levels. Assay plates were incubated at 37 °C for 2 h followed by measurement of 450 nm and 650 nm luminescence (80 nm bandwidth) at room temperature with a CLARIOstar (BMG Labtech) plate reader 2 min after NanoLuc substrate addition.

BRET ratios were calculated from 650 nm/450 nm luminescence and normalized for each plate using 50 µM SAHA treated negative controls and uninhibited positive controls. pIC₅₀-values were calculated from normalized BRET ratios using nonlinear regression log(inhibitor) four parameters least squares fit in GraphPad Prism version 7.04 for Windows, GraphPad Software, La Jolla California USA, www.graphpad.com.

### HDAC2 Assay:

HDAC2 inhibition was tested using the HDAC-Glo™ I/II Assay and Screening System (G6421, Promega) with recombinant HDAC2 (50002, BPS Bioscience). The assay was carried out in a 384-well plate (4512, Corning) format using 10 ng/mL (0.18 nM) HDAC2 enzyme according to the manufacturer's description. Inhibitors were tested at eight 1:4.12 serial dilutions in triplicates ranging from 8,67 nM to 200 µM. Drug dosing was performed from 10 mM DMSO stock solutions with a D300e Digital Dispenser (Tecan). Note: Due to the dosing increments of the drug printer, the dilution factor is not entirely stable over all dose levels. HDAC2 and inhibitors were incubated together for 30 min at room temperature. After addition of the HDAC-Glo™ I/II reagent, plates were shaken (800 rpm orbital shaker, 30 s), centrifuged (300 g, 1 min) and incubated at room temperature for 40 min. Luminescence was detected with a CLARIOstar (BMG Labtech) plate reader. Luminescence signal was normalized with 200 µM SAHA treated negative controls and uninhibited positive controls. pIC₅₀-values were then calculated as described in the BRET assay.

**Table 1**

| Compound | | HDAC10 pIC50 | HDAC6 pIC50 | HDAC2 pIC50 |
|---|---|---|---|---|
| **SAHA** | 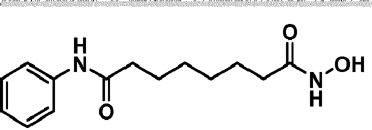 | 6.2 | 6.9 | 6.2 |
| **DKFZ-621** | 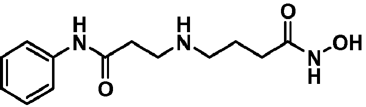 | 6.1 | <4.4 | nd |
| **DKFZ-711** | 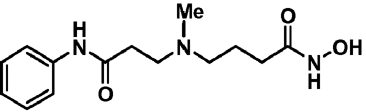 | 6.7 | ∼4.4 | ∼3.7 |
| **DKFZ-728** | 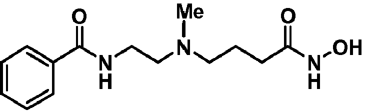 | 6.9 | <4.4 | <3.7 |

**Table 2**

| | | | | |
|---|---|---|---|---|
| 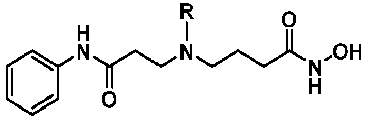 | | | | |

| Compound | **R** | HDAC10 pIC50 | HDAC6 pIC50 | HDAC2 pIC50 |
|---|---|---|---|---|
| **SAHA** | --- | 6.2 | 6.9 | 6.2 |
| **DKFZ-711** | 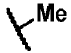 | 6.7 | ∼4.4 | ∼3.7 |
| **DKFZ-714** | 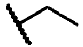 | 6.1 | <4.4 | <3.7 |
| **DKFZ-716** | 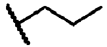 | 6.0 | <4.4 | <3.7 |
| **DKFZ-715** | 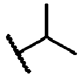 | 5.7 | <4.4 | <3.7 |
| **DKFZ-724** | 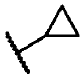 | 6.4 | <4.4 | <3.7 |
| **DKFZ-717** | 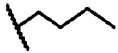 | 5.8 | <4.4 | <3.7 |
| **DKFZ-718** | 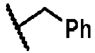 | 6.3 | ∼4.9 | ∼3.8 |

### REFERENCES

(1) Taunton, J., et al. A mammalian histone deacetylase related to the yeast transcriptional regulator Rpd3p. Science 1996, 272, 408-411.
(2) Marks, P. A.; Breslow, R. Dimethyl sulfoxide to vorinostat: development of this histone deacetylase inhibitor as an anticancer drug. Nat. Biotechnol. 2007, 25, 84-90.
(3) Falkenberg, K. J.; Johnstone, R. W. Histone deacetylases and their inhibitors in cancer, neurological diseases and immune disorders. Nat. Rev. Drug Disc. 2014, 13, 673-691.
(4) Roche, J.; Bertrand, P. Inside HDACs with more selective HDAC inhibitors. Eur. J. Med. Chem. 2016, 121, 451-483.
(5) Kutil, Z., et al. Histone deacetylase 11 is a fatty-acid deacylase. ACS Chem. Biol. 2018, 13, 685-693.
(6) Hai, Y., et al. Histone deacetylase 10 structure and molecular function as a polyamine deacetylase. Nat. Commun. 2017, 8, 15368.
(7) Aramsangtienchai, P., et al. HDAC8 catalyzes the hydrolysis of long chain fatty acyl lysine. ACS Chem. Biol. 2016, 11, 2685-2692.
(8) Feldman, J. L., et al. Activation of the protein deacetylase SIRT6 by long-chain fatty acids and widespread deacylation by mammalian sirtuins. J. Biol. Chem. 2013, 288, 31350-31356.
(9) Moreno-Yruela, C., et al. Histone Deacetylase 11 Is an ε-N-Myristoyllysine Hydrolase. Cell Chem. Biol. 2018, 25, 849-856.
(10) Witt, O., et al. HDAC family: What are the cancer relevant targets? Cancer Lett. 2009, 277, 8-21*.*
(11) Balasubramanian, S., et al. Isoform-specific histone deacetylase inhibitors: The next step? Cancer Lett. 2009, 280, 211-221.
(12) Bradner, J. E., et al. Chemical phylogenetics of histone deacetylases. Nat. Chem. Biol. 2010, 6, 238-243.
(13) Boskovic, Z. V., et al. Inhibition of zinc-dependent histone deacetylases with a chemically triggered electrophile. ACS Chem. Biol. 2016, 11, 1844-1851.
(14) Lobera, M., et al. Selective class IIa histone deacetylase inhibition via a nonchelating zinc-binding group. Nat. Chem. Biol. 2013, 9, 319-325.
(15) Mai, A., et al. Class II (IIa)-selective histone deacetylase inhibitors. 1. Synthesis and biological evaluation of novel (aryloxopropenyl)pyrrolyl hydroxyamides. J. Med. Chem. 2005, 48, 3344-3353.
(16) Martin, M. W., et al. Discovery of novel N-hydroxy-2-arylisoindoline-4-carboxamides as potent and selective inhibitors of HDAC11. Bioorg. Med. Chem. Lett. 2018, 28, 2143-2147.
(17) Marek, L., et al. Histone deacetylase (HDAC) inhibitors with a novel connecting unit linker region reveal a selectivity profile for HDAC4 and HDAC5 with improved activity against chemoresistant cancer cells. J. Med. Chem. 2013, 56, 427-436.
(18) Haggarty, S. J., et al. Domain-selective small-molecule inhibitor of histone deacetylase 6 (HDAC6)-mediated tubulin deacetylation. Proc. Nat. Acad. Sci. 2003, 100, 4389-4394.
(19) Butler, K. V., et al. Rational design and simple chemistry yield a superior, neuroprotective HDAC6 inhibitor, tubastatin A. J. Am. Chem. Soc. 2010, 132, 10842-10846.
(20) Jochems, J., et al. Antidepressant-like properties of novel HDAC6-selective inhibitors with improved brain bioavailability. Neuropsychopharmacology 2013, 39, 389-400.
(21) Arrowsmith, C. H., et al. The promise and peril of chemical probes. Nat. Chem. Bio. 2015, 11, 536-541.
(22) Fischer, D. D., et al. Isolation and characterization of a novel class II histone deacetylase, HDAC10. J. Biol. Chem. 2002, 277, 6656-6666.
(23) Guardiola, A. R.; Yao, T. P. Molecular cloning and characterization of a novel histone deacetylase HDAC10. J. Biol. Chem. 2002, 277, 3350-3356.
(24) Kao, H. Y., et al. Isolation and characterization of mammalian HDAC10, a novel histone deacetylase. J. Biol. Chem. 2002, 277, 187-193.
(25) Lai, I. L., et al. Histone deacetylase 10 relieves repression on the melanogenic program by maintaining the deacetylation status of repressors. J. Biol. Chem. 2010, 285, 7187-7196.
(26) Park, B. L., et al. HDAC10 promoter polymorphism associated with development of HCC among chronic HBV patients. Biochem. Biophys. Res. Commun. 2007, 363, 776-781.
(27) Kotian, S., et al. Histone deacetylases 9 and 10 are required for homologous recombination. J. Biol. Chem. 2011, 286, 7722-7726.
(28) Park, J. H., et al. Class II histone deacetylases play pivotal roles in heat shock protein 90-mediated proteasomal degradation of vascular endothelial growth factor receptors. Biochem. Biophys. Res. Commun. 2008, 368, 318-322.
(29) Jin, Z., et al. Decreased expression of histone deacetylase 10 predicts poor prognosis of gastric cancer patients. Int. J. Clin. Exp. Pathol. 2014, 7, 5872-5879.
(30) Osada, H., et al. Reduced expression of class II histone deacetylase genes is associated with poor prognosis in lung cancer patients. International journal of cancer 2004, 112, 26-32.
(31) Powers, J., et al. Expression and function of histone deacetylase 10 (HDAC10) in B cell malignancies. Methods in molecular biology 2016, 1436, 129-145.
(32) Song, C., et al. Histone deacetylase (HDAC) 10 suppresses cervical cancer metastasis through inhibition of matrix metalloproteinase (MMP) 2 and 9 expression. J. Biol. Chem. 2013, 288, 28021-28033.
(33) Fan, W., et al. Histone deacetylase 10 suppresses proliferation and invasion by inhibiting the phosphorylation of beta-catenin and serves as an independent prognostic factor for human clear cell renal cell carcinoma. Int. J. Clin. Exp. Med. 2015, 8, 3734-3742.
(34) Islam, M. M., et al. HDAC10 as a potential therapeutic target in ovarian cancer. Gynecol. Oncol. 2017, 144, 613-620.
(35) Oehme, I., et al. Histone deacetylase 10-promoted autophagy as a druggable point of interference to improve the treatment response of advanced neuroblastomas. Autophagy 2013, 9, 2163-2165.
(36) Yang, Y., et al. HDAC10 promotes lung cancer proliferation via AKT phosphorylation. Oncotarget 2016, 7, 59388-59401.
(37) Oehme, I., et al. Histone deacetylase 10 promotes autophagy-mediated cell survival. Proc. Nat. Acad. Sci. 2013, 110, E2592-E2601.
(38) Ridinger, J., et al. Dual role of HDAC10 in lysosomal exocytosis and DNA repair promotes neuroblastoma chemoresistance. Sci. Rep. 2018, 8, 10039.
(39) Bantscheff, M., et al. Chemoproteomics profiling of HDAC inhibitors reveals selective targeting of HDAC complexes. Nat. Biotechnol. 2011, 29, 255-265.
(40) Kolbinger, F. R., et al. The HDAC6/8/10 inhibitor TH34 induces DNA damage-mediated cell death in human high-grade neuroblastoma cell lines. Arch.Toxicol. 2018, 92, 2649 2664.
(41) Géraldy, M., et al. Selective inhibition of histone deacetylase 10: Hydrogen bonding to the gatekeeper residue is implicated. ChemRxiv preprint; DOI: 10.26434/chemrxiv.7406618.v1
(42) Wang, M., et al. A Novel Role for Histone Deacetylase 10 (HDAC10) in the Regulation of PD-L1 Expression and Immune Tolerance Mediated By Antigen Presenting Cells (APCs). Blood 2017 130:3561.

## Claims

1. An HDAC10 inhibitor of Formula (**I**)
CAP-(CR^{y}R^{y'})ₙ-NR²-CR³R^{3'}-CR⁴R^{4'}-CR⁵R^{5'}-ZBD (**I**)
wherein:
CAP is a capping group selected from the group of aryl-X- and heteroaryl-X-, wherein X is absent or is selected from -C(=O)-NR¹-, -NR-C(=O)-, -S(=O)₂-NR¹-,-NR-S(=O)₂-, -S(=O)(=NR)-NR¹-, -NR-S(=O)(=NR)-, -C(=NR)-, -O-, -S-, -S(=O)-, - S(=O)₂-, and -C(=O)-,
wherein
R and R¹ are independently a residue selected from H and a substituent selected from linear or branched C₁₋₄-alkyl, cyclopropyl, benzyl, aryl and heteroaryl, wherein said substituent is optionally further substituted, in particular by a further substituent selected from the list of -F, -OH, -OR, and -NR₂;
n is an integer selected from 1, 2 and 3;
y is an integer taking the values from the range of 1 to n;
ZBD is a zinc-binding domain selected from the group of:
-C(=O)-NH-OH, -C(=N-OH)-C(=O)NH-R⁶, -C(=O)CF₃, -C(=O)-NH-C₆H₄-o-NH₂,-C(=O)CH₂SH, -SH, -C(=NH)-NH-OH, and -C(=N-OH)-NH₂, wherein R⁶ is a residue selected from H, linear or branched C₁₋₄-alkyl,-cyclopropyl, and benzyl;
and
R² is a residue selected from H and a substituent selected from linear or branched C₁₋₄-alkyl, cyclopropyl, benzyl, aryl and heteroaryl, wherein said substituent is optionally further substituted, in particular by a further substituent selected from the list of -F, -OH, -OR, and -NR₂, and R¹, each R^{y}, each R^{y'}, R³, R^{3'}, R⁴, R^{4'}, R⁵, and R^{5'} are independently selected from residues H, CH₃, F, CFH₂, CF₂H and CF₃, provided that in total not more than three of said residues are different from -H.

2. The HDAC10 inhibitor of claim 1, wherein CAP is a capping group selected from the group of: aryl-C(=O)-NH-, heteroaryl-C(=O)-NH-, and aryl-NH-C(=O)-, and heteroaryl-NH-C(=O)-.

3. The HDAC10 inhibitor of claim 2, wherein CAP is phenyl-NH-C(=O)- or phenyl-C(=O)-NH-, particularly phenyl-NH-C(=O)-.

4. The HDAC10 inhibitor of any one of claims 1 to 3, wherein n is 2.

5. The HDAC10 inhibitor of any one of claims 1 to 4, wherein R² is a residue selected from H, methyl, ethyl, n-propyl, i-propyl, n-butyl, cyclopropyl, and benzyl.

6. The HDAC10 inhibitor of any one of claims 1 to 5, wherein each R^{y}, each R^{y'}, R³, R^{3'}, R⁴, R^{4'}, R⁵, and R^{5'} are each -H.

7. The HDAC10 inhibitor of any one of claims 1 to 6, wherein ZBD is -C(=O)-NH-OH.

8. The HDAC10 inhibitor of any one of claims 1 to 7, wherein the HDAC10 inhibitor is selected from the group of DKFZ711, DKFZ714, DKFZ715, DKFZ716, DKFZ717, DKFZ718, DKFZ724 and DKFZ728.

9. A pharmaceutically acceptable salt form of the HDAC10 inhibitor of any one of claims 1 to 8.

10. The pharmaceutically acceptable salt form of claim 9, wherein the HDAC10 of any one of claims 1 to 8 is reacted with an acid selected from the group of: hydrochloric, hydrobromic, sulfuric, sulfamic, phosphoric, nitric; acetic, propionic, succinic, glycolic, stearic, lactic, malic, tartaric, citric, ascorbic, palmoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic acid, in particular hydrochloric acid.

11. A pharmaceutical composition comprising an HDAC10 inhibitor of any one of claims 1 to 8, or a pharmaceutically acceptable salt form of an HDAC10 inhibitor of claim 9 or 10.

12. An HDAC10 inhibitor of any one of claims 1 to 8, a pharmaceutically acceptable salt form of an HDAC10 inhibitor of claim 9 or 10, or the pharmaceutical composition of claim 11 for use in the treatment of cancer.

13. An HDAC10 inhibitor of any one of claims 1 to 8, a pharmaceutically acceptable salt form of an HDAC10 inhibitor of claim 9 or 10, or a pharmaceutical composition of claim 11 for the use of claim 12, wherein autophagy is upregulated in the cells of said cancer.
